# EUROPÄISCHE PATENTSCHRIFT

(11) **EP 0 663 390 B1**
(45) Veröffentlichungstag und Bekanntmachung des Hinweises auf die Patenterteilung: **12.02.1997**
(21) Anmeldenummer: 94118414.5
(22) Anmeldetag: 23.11.1994
(51) Int. Cl.: C07C 231/02

(54) **Verfahren zur Herstellung von aromatischen Carbonsäurenamiden aus aromatischen Carbonsäuren und Harnstoff**
Process for the preparation of aromatic amides form aromatic carboxylic acids and urea
Procédé pour la préparation d'amides aromatiques à partir d'acides carboxyliques aromatiques et urée

(30) Priorität: 14.12.1993 DE 4342571
(43) Veröffentlichungstag der Anmeldung: 19.07.1995
(73) Patentinhaber: HOECHST AKTIENGESELLSCHAFT, 65929 Frankfurt am Main (DE)
(72) Erfinder: Christidis, Yani, Dr., F-75010 Paris (FR); Meier, Michael, Dr., D-60487 Frankfurt am Main (DE)

(56) Entgegenhaltungen:
- EGYPTIAN JOURNAL OF PHARMACEUTICAL SCIENCES, Bd. 13,Nr. 2, 1972 CAIRO, Seiten 231-235, A.I. EL-SEBAL 'STUDY OF FUSION OF UREA WITH SOME AROMATIC ACIDS "NEW FINDINGS"'
- CHEMICAL ABSTRACTS, vol. 103, no. 3, 1985 Columbus, Ohio, US; abstract no. 22297z, TORAY INDUSTRIES, INC. 'BENZONITRILES' Seite 548;

## Beschreibung

Die Erfindung betrifft ein Verfahren zur Herstellung von aromatischen Carbonsäureamiden aus aromatischen Carbonsäuren und Harnstoff in einem inerten organischen Lösungsmittel unter Zusatz von Phosphorigsäure.

Aromatische Carbonsäureamide sind wichtige Zwischenprodukte für Farbstoffe, Pigmente, Pharmazeutika und Pflanzenschutzmittel.

Die Umsetzung von Carbonsäuren mit äquimolaren Mengen Harnstoff in der Schmelze bei 200 - 230°C zu den entsprechenden Carbonsäureamiden ist in Helv. Chim. Acta 29, 1438 - 1446 (1946) beschrieben. Benzamid wird hierbei in einer Ausbeute von 60 % erhalten.

Die Herstellung von 2-Hydroxynaphthalin-3-carbonsäureamid und Salicylsäureamid aus den Carbonsäuren mit 4 mol Harnstoff in der Schmelze unter Zusatz einer katalytischen Menge Phosphorsäure bei 170 bis 180°C in 58 bzw. 73 % Ausbeute isk bekannt.

Die Herstellung von p-Hydroxybenzoesäureamid aus p-Hydroxybenzoesäure und 2 bis 3 mol Harnstoff in einem inerten organischen Lösungsmittel mit einem Siedepunkt > 160°C, bevorzugt 180 bis 230°C und Zusatz stöchiometrischer Mengen Amidosulfonsäure wird in der JP 5 043 531 beschrieben. Nachteilig an diesem Verfahren sind der sehr hohe Überschuß an Harnstoff und die Verwendung stöchiometrischer Mengen Amidosulfonsäure. Die Ausbeute beträgt 76,6 %.

Ebenso ist die Umsetzung von aromatischen Carbonsäuren mit leichtem Überschuß Harnstoff bei 200 bis 220°C bekannt. Hierbei entstehen in 41 bis 94 % Rohausbeute die entsprechenden Amide, die zur Erzielung brauchbarer Qualitäten umkristallisiert werden müssen (Chem. Listy 46, 306 (1952)).

Allen Verfahren in der Schmelze ist gemeinsam, daß die entstehenden Carbonsäureamide stark gefärbt und verunreinigt sind. Als Nebenprodukte entstehen bis zu ca. 25 % Nitrile und bis zu 10 % der entsprechenden Triazine (Egypt. J. Pharm. Sci. 13, 231 (1972)). Außerdem sind die Schmelzen auf Grund der hohen Erstarrungspunkte technisch nur schwer zu handhaben.

Es besteht daher ein großer Bedarf nach einem technisch leicht durchführbaren Verfahren zur Herstellung von aromatischen Carbonsäureamiden aus aromatischen Carbonsäuren mit Harnstoff, das die vorstehend genannten Nachteile vermeidet und aromatische Carbonsäureamide nicht nur in hoher Reinheit, sondern auch in hoher Ausbeute liefert.

Diese Aufgabe wird gelöst durch ein Verfahren zur Herstellung von aromatischen Carbonsäureamiden der allgemeinen Formel (I) 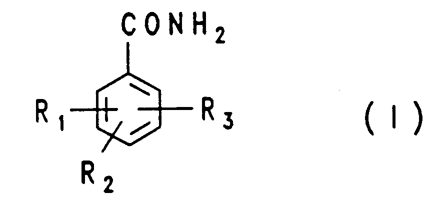 in welcher R₁, R₂ und R₃ gleich oder verschieden sind und Wasserstoff-, Fluor-, Chlor-, Bromatome, Alkyl(C₁-C₄)-, Hydroxy- oder Nitrogruppen bedeuten, oder R₁ und R₂ einen aromatischen Ring mit 5 oder 6 Ringgliedern bilden, welcher durch Fluor-, Chlor-, Bromatome, Alkyl(C₁-C₄)-, Hydroxy- oder Nitrogruppen substituiert sein kann.

Es ist dadurch gekennzeichnet, daß man aromatische Carbonsäuren der allgemeinen Formel (II), 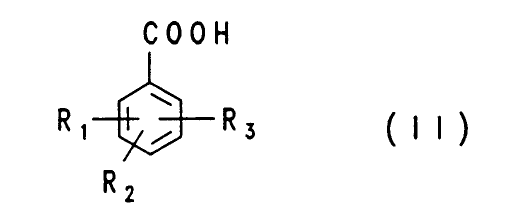 in welcher R₁, R₂ und R₃ die vorstehend genannten Bedeutungen haben, in einem inerten organischen Lösungsmittel mit Harnstoff unter Zusatz von einer katalytischen Menge Phosphorigsäure umsetzt.

Von besonderem Interesse ist das Verfahren zur Herstellung von Verbindungen der Formel (I), in der R₁, R₂ und R₃ gleich oder verschieden sind und Fluor-, Chlor-, Wasserstoffatome oder Nitrogruppen bedeuten.

Bei dem erfindungsgemäßen Verfahren wird als inertes organisches Lösungsmittel ein aromatischer Kohlenwasserstoff, ein chlorierter aromatischer Kohlenwasserstoff oder Mischungen derselben eingesetzt. In vielen Fällen hat es sich bewährt, Cumol, Mesitylen, Cymol, Diisopropylnaphtalin, ein Chlortoluol und/oder Dichlorbenzol in etwa 1 bis 20, bevorzugt 2 bis 5 Gewichtsteilen je Gewichtsteil aromatisches Carbonsäureamid zu verwenden. An Stelle des reinen Lösungsmittels kann die Mutterlauge aus einem vorhergehenden Ansatz ohne weitere Reinigung eingesetzt werden. Selbstverständlich lassen sich auch größere Mengen Lösungsmittel verwenden, was jedoch die Raumzeitausbeute herabsetzt.

Es hat sich als vorteilhaft erwiesen, in die Reaktion je mol aromatischem Carbonsäureamid 0,8 bis 1,5 mol, insbesondere 1,0 bis 1,3 mol Harnstoff einzusetzen.

Es hat sich bewährt, Phosphorigsäure als Katalysator in Mengen von 0,01 bis 5, bevorzugt 0,05 bis 2 Gew.-% der Reaktion zuzusetzen.

Weiterhin hat sich als nützlich erwiesen, die Umsetzung bei Temperaturen von 140 bis 200°C, insbesondere bei Temperaturen von 150 bis 180°C durchzuführen.

Die Selektivität in Bezug auf die Carbonsäure ist in der Regel größer als 98 % und die in der Literatur (Egypt. J. Pharm. Sci. 13, 231 (1972)) beschriebenen Nebenprodukte werden kaum gebildet. Nach dem erfindungsgemäßen Verfahren werden aromatische Carbonsäureamide in der Regel mit Ausbeuten über 80 % in hoher Reinheit gebildet. Nicht umgesetzte Carbonsäure kann zurückgewonnen und in einem neuen Ansatz wieder eingesetzt werden. An Stelle des reinen Lösungsmittels kann die Mutterlauge aus einem vorhergehenden Ansatz ohne weitere Reinigung eingesetzt werden. Dadurch kann die Ausbeute in vielen Fällen auf ca. 95 % gesteigert werden.

Die nachstehenden Beispiele dienen der Erläuterung des erfindungsgemäßen Verfahrens, ohne es zu beschränken.

### Beispiel 1: 4-Nitrobenzoesäureamid

83,5 g (0,5 mol) 4-Nitrobenzoesäure, 33,0 g (0,55 mol) Harnstoff und 0,5 g Phosphorigsäure werden in 250 ml Diisopropylnaphthalin unter Rühren 8 h auf 150 bis 158°C erhitzt. Nach dem Abkühlen auf 25°C wird abgesaugt und mit 50 ml Diisopropylnaphthalin gewaschen. Der Filterkuchen wird in 250 ml Wasser eingetragen, mit Natronlauge pH 12 eingestellt und 30 min bei 25°C gerührt. Anschließend wird abgesaugt und bei 100°C getrocknet. Man erhält 81,4 g 4-Nitrobenzoesäureamid mit einem Schmelzpunkt von 191 bis 194°C. Dies entspricht einer Ausbeute von 98 % d.Th..

### Beispiel 2: 4-Nitrobenzoesäureamid

83,5 g (0,5 mol) 4-Nitrobenzoesäure, 33,0 g (0,55 mol) Harnstoff und 0,5 g Phosphorigsäure werden in 250 ml o-Chlortoluol unter Rühren 8 h auf 150 bis 157°C erhitzt. Nach dem Abkühlen auf 25°C wird abgesaugt und mit 50 ml o-Chlortoluol gewaschen. Der Filterkuchen wird in 250 ml Wasser eingetragen, mit Natronlauge pH 12 gestellt und wasserdampfdestilliert. Anschließend wird abgesaugt und bei 100°C getrocknet. Man erhält 74,9 g 4-Nitrobenzoesäureamid mit einem Schmelzpunkt von 194 bis 195°C. Dies entspricht einer Ausbeute von 90,3 % d.Th.. Durch Ansäuern der wäßrigen Mutterlauge auf pH 2 werden 3,4 g 4-Nitrobenzoesäure (4,1 % d.Th.) erhalten, die in einem Folgeansatz wieder eingesetzt werden können.

### Beispiel 3: 4-Nitrobenzoesäureamid

83,5 g (0,5 mol) 4-Nitrobenzoesäure, 33,0 g (0,55 mol) Harnstoff und 0,5 g Phosphorigsäure werden in 250 ml Cumol unter Rühren 8 h auf 150°C erhitzt. Nach dem Abkühlen auf 25°C wird abgesaugt und mit 50 ml Cumol gewaschen. Der Filterkuchen wird in 250 ml Wasser eingetragen, mit Natronlauge pH 12 eingestellt und wasserdampfdestilliert. Anschließend wird abgesaugt und bei 100°C getrocknet. Man erhält 71,3 g 4-Nitrobenzoesäureamid mit einem Schmelzpunkt von 192 bis 193°C. Dies entspricht einer Ausbeute von 85,9 % d.Th.. Durch Ansäuern der wäßrigen Mutterlauge auf pH 2 werden 4,9 g 4-Nitrobenzoesäure (5,9 % d.Th.) erhalten, die in einem Folgeansatz wieder eingesetzt werden können.

### Beispiel 4: 4-Nitrobenzoesäureamid

334 g (2,0 mol) 4-Nitrobenzoesäure, 120 g (2,0 mol) Harnstoff und 2,0 g Phosphorigsäure werden in 1000 ml Mesitylen unter Rühren 8 h auf 158 bis 163°C erhitzt. Nach dem Abkühlen auf 25°C wird abgesaugt und mit 200 ml Mesitylen gewaschen. Der Filterkuchen wird in 1000 ml Wasser eingetragen, mit Natronlauge pH 12 eingestellt und wasserdampfdestilliert. Anschließend wird abgesaugt und bei 100°C getrocknet. Man erhält 301,2 g 4-Nitrobenzoesäureamid mit einem Schmelzpunkt von 191 bis 194°C. Dies entspricht einer Ausbeute von 90,7 % d.Th.. Durch Ansäuern der wäßrigen Mutterlauge auf pH 2 werden 12,5 g 4-Nitrobenzoesäure (3,7 % d.Th.) erhalten, die in einem Folgeansatz wieder eingesetzt werden können.

334 g (2,0 mol) 4-Nitrobenzoesäure, 120 g (2,0 mol) Harnstoff und 2,0 g Phosphorigsäure werden in 1000 ml organischer Mutterlauge aus dem vorhergehenden Versuch unter Rühren auf 158 bis 163°C erhitzt. Die Aufarbeitung erfolgt wie oben beschrieben. Man erhält 313,4 g 4-Nitrobenzoesäureamid mit einem Schmelzpunkt von 192 bis 195°C. Dies entspricht einer Ausbeute von 94,4 % d.Th.. Durch Ansäuern der wäßrigen Mutterlauge auf pH 2 werden 8,8 g 4-Nitrobenzoesäure (2,6 % d.Th.) erhalten.

### Beispiel 5: 4-Chlor-3-nitrobenzoesäureamid

100 g (0,5 mol) 4-Chlor-3-nitrobenzoesäure, 36 g (0,6 mol) Harnstoff und 0,5 g Phosphorigsäure werden in 250 ml 1,2-Dichlorbenzol unter Rühren 8 h auf 165 bis 178°C erhitzt. Nach dem Abkühlen auf 25°C wird abgesaugt und mit 50 ml 1,2-Dichlorbenzol gewaschen. Der Filterkuchen wird in 250 ml Wasser eingetragen, mit Natronlauge pH 12 eingestellt und wasserdampfdestilliert. Anschließend wird abgesaugt und bei 100°C getrocknet. Man erhält 89,7 4-Chlor-3-nitrobenzoesäureamid mit einem Schmelzpunkt von 146 bis 149°C. Dies entspricht einer Ausbeute von 90,2 % d.Th.. Durch Ansäuern der wäßrigen Mutterlauge auf pH 2 werden 4,9 g 4-Chlor-3-nitrobenzoesäure (4,9 % d.Th.) erhalten, die in einem Folgeansatz wieder eingesetzt werden können.

### Beispiel 6: Benzoesäureamid

61,0 g (0,5 mol) Benzoesäure, 30,0 g (0,5 mol) Harnstoff und 0,5 g Phosphorigsäure werden in 100 ml Mesitylen unter Rühren 8 h auf 150 bis 158°C erhitzt. Nach dem Abkühlen auf 25°C wird abgesaugt und mit 30 ml Mesitylen gewaschen. Der Filterkuchen wird in 100 ml Wasser eingetragen, mit Natronlauge pH 12 eingestellt und wasserdampfdestilliert. Anschließend wird abgesaugt und bei 100°C getrocknet. Man erhält 51,1 g Benzoesäure mit einem Schmelzpunkt von 122 bis 125°C. Dies entspricht einer Ausbeute von 85,2 % d.Th..

### Beispiel 7: 4-Fluorbenzoesäureamid

70,0 g (0,5 mol) 4-Fluorbenzoesäure, 30,0 g (0,50 mol) Harnstoff und 0,5 g Phosphorigsäure werden in 100 ml Mesitylen unter Rühren 8 h auf 150 bis 158°C erhitzt. Nach dem Abkühlen auf 25°C wird abgesaugt und mit 50 ml Mesitylen gewaschen. Der Filterkuchen wird in 250 ml Wasser eingetragen, mit Natronlauge pH 12 eingestellt und wasserdampfdestilliert. Anschließend wird abgesaugt und bei 100°C getrocknet. Man erhält 50,7 g 4-Fluorbenzoesäureamid mit einem Schmelzpunkt von 147 bis 150°C. Dies entspricht einer Ausbeute von 72,9 % d.Th.. Durch Ansäuern der wäßrigen Mutterlauge auf pH 2 werden 5,9 g 4-Fluorbenzoesäure (8,4 % d.Th.) erhalten, die in einem Folgeansatz wieder eingesetzt werden können.

### Beispiel 8: Isophthalsäurediamid

83,0 g (0,5 mol) Isophthalsäure, 60,0 g (1,0 mol) Harnstoff und 0,5 g Phosphorigsäure werden in 250 ml Mesitylen unter Rühren 8 h auf 155 bis 158°C erhitzt. Nach dem Abkühlen auf 25°C wird abgesaugt und mit 50 ml Mesitylen gewaschen. Nach üblicher Aufarbeitung erhalt man 60,3 g Isophthalsäurediamid mit einem Schmelzpunkt von 291 bis 293°C. Dies entspricht einer Ausbeute von 73,5 % d.Th..

### Beispiel 9: 4-Nitrobenzoesäureamid (Vergleichsbeispiel ohne Phosphorigsäure)

41,8 g (0,25 mol) 4-Nitrobenzoesäure und 15,0 g (0,25 mol) Harnstoff werden in 75 ml Mesitylen unter Rühren 8 h auf 158 bis 163°C erhitzt. Nach dem Abkühlen auf 25°C wird abgesaugt und mit 25 ml Mesitylen gewaschen. Der Filterkuchen wird in 100 ml Wasser eingetragen, mit Natronlauge pH 12 eingestellt und wasserdampfdestilliert. Anschließend wird abgesaugt und bei 100°C getrocknet. Man erhält 4,8 g 4-Nitrobenzoesäureamid mit einem Schmelzpunkt von 191 bis 194°C. Dies entspricht einer Ausbeute von 11,6 % d.Th..

### Beispiel 10: 4-Nitrobenzoesäureamid (Vergleichsbeispiel mit Phosphorsaure)

83,5 g (0,50 mol) 4-Nitrobenzoesäure, 30,0 g (0,50 mol) Harnstoff und 1 g Phosphorsäure werden in 250 ml Mesitylen unter Rühren 8 h auf 160 bis 162°C erhitzt. Nach dem Abkühlen auf 25°C wird abgesaugt und mit 25 mo Mesitylen gewaschen. Der Filterkuchen wird in 250 ml Wasser eingetragen, mit Natronlauge pH 12 eingestellt und wasserdampfdestilliert. Anschließend wird abgesaugt und bei 100°C getrocknet. Man erhält 52,0 g 4-Nitrobenzoesäureamid mit einem Schmelzpunkt von 193 bis 195°C. Dies entspricht einer Ausbeute von 62,7 % d.Th..

### Beispiel 11: 4-Nitrobenzoesäureamid (Vergleichsbeispiel mit Amidosulfonsäure)

83,5 g (0,50 mol) 4-Nitrobenzoesäure, 30,0 g (0,50 mol) Harnstoff und 0,5 g Amidosulfonsäure werden in 200 ml Dichlorbenzol unter Rühren 16 h auf 180 bis 181°C erhitzt. Nach dem Abkühlen auf 25°C wird abgesaugt und mit 50 ml Dichlorbenzol gewaschen. Der Filterkuchen wird in 250 ml Wasser eingetragen, mit Natronlauge pH 12 eingestellt und wasserdampfdestilliert. Anschließend wird abgesaugt und bei 100°C getrocknet. Man erhält 40,6 g 4-Nitrobenzoesäureamid mit einem Schmelzpunkt von 192 bis 195°C. Dies entspricht einer Ausbeute von 48,9 % d.Th..

## Patentansprüche

1. Verfahren zur Herstellung von aromatischen Carbonsäureamiden der allgemeinen Formel (I) 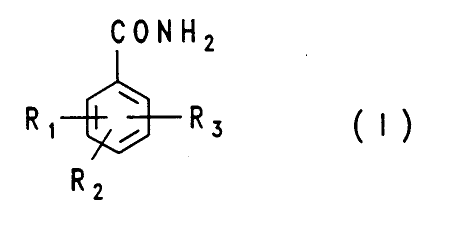 in welcher R₁, R₂ und R₃ gleich oder verschieden sind und Wasserstoff-, Fluor-, Chlor-, Bromatome, Alkyl(C₁-C₄)-, Hydroxy- oder Nitrogruppen bedeuten, oder R₁ und R₂ einen aromatischen Ring mit 5 oder 6 Ringgliedern bilden, welcher durch Fluor-, Chlor-, Bromatome, Alkyl(C₁-C₄)-, Hydroxy- oder Nitrogruppen substituiert sein kann, dadurch gekennzeichnet, daß man aromatische Carbonsäuren der allgemeinen Formel (II), 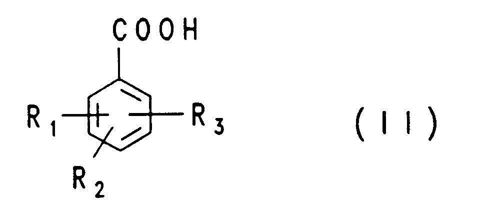 in welcher R₁, R₂ und R₃ die vorstehend genannten Bedeutungen haben, in einem inerten organischen Lösungsmittel mit Harnstoff unter Zusatz einer katalytischen Menge Phosphorigsäure umsetzt.

2. Verfahren nach Anspruch 1, dadurch gekennzeichnet, daß R₁, R₂, R₃ gleich oder verschieden sind und Fluor-, Chlor-, Wasserstoffatome oder Nitrogruppen bedeuten.

3. Verfahren nach Anspruch 1 oder 2, dadurch gekennzeichnet, daß man als inertes organisches Lösungsmittel einen aromatischen Kohlenwasserstoff, einen chlorierten aromatischen Kohlenwasserstoff oder Mischungen derselben einsetzt.

4. Verfahren nach einem oder mehreren der Ansprüche 1 bis 3, dadurch gekennzeichnet, daß man als inertes organisches Lösungsmittel Cumol, Mesitylen, Cymol, Diisopropylnaphtalin, ein Chlortoluol und/oder Dichlorbenzol einsetzt.

5. Verfahren nach einem oder mehreren der Ansprüche 1 bis 4, dadurch gekennzeichnet, daß man je Gewichtsteil aromatische Carbonsäure 1 bis 20, bevorzugt 2 bis 5 Gewichtsteile Lösungsmittel einsetzt.

6. Verfahren nach einem oder mehreren der Ansprüche 1 bis 5, dadurch gekennzeichnet, daß man je mol aromatischem Carbonsäureamid 0,8 bis 1,5 mol, insbesondere 1,0 bis 1,3 mol Harnstoff einsetzt.

7. Verfahren nach einem oder mehreren der Ansprüche 1 bis 6, dadurch gekennzeichnet, daß man Phosphorigsäure als Katalysator in Mengen von 0,01 bis 5, bevorzugt 0,05 bis 2 Gew.-% der Reaktion zusetzt.

8. Verfahren nach einem oder mehreren der Ansprüche 1 bis 7, dadurch gekennzeichnet, daß man die Umsetzung bei Temperaturen von 140 bis 200°C, insbesondere bei Temperaturen von 150 bis 170°C durchführt.

9. Verfahren nach einem oder mehreren der Ansprüche 1 bis 8, dadurch gekennzeichnet, daß man an Stelle des reinen Lösungsmittels die Mutterlauge aus einem vorhergehenden Ansatz einsetzt.

10. Verfahren nach einem oder mehreren der Ansprüche 1 bis 9, dadurch gekennzeichnet, daß man nicht umgesetzte Carbonsäure zurückgewinnt und in die Reaktion zurückführt.

## Claims

1. A process for the preparation of an aromatic carboxamide of the formula (I) 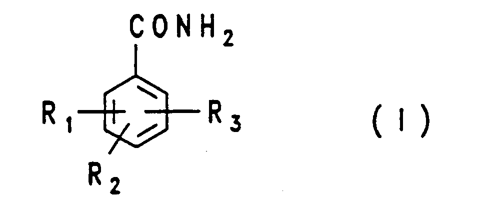 in which R₁, R₂ and R₃ are identical or different and are hydrogen, fluorine, chlorine or bromine atoms, or are alkyl(C₁-C₄), hydroxyl or nitro groups, or R₁ and R₂ form an aromatic ring of 5 or 6 ring members, which ring may be substituted by fluorine, chlorine or bromine atoms or by alkyl(C₁-C₄), hydroxyl or nitro groups, which comprises reacting an aromatic carboxylic acid of the formula (II) 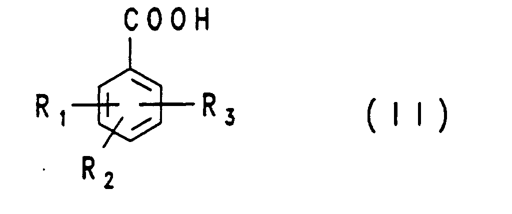 in which R₁, R₂ and R₃ are as defined above with urea in an inert organic solvent with the addition of a catalytic count of phosphorous acid.

2. The process as claimed in claim 1, wherein R₁, R₂ and R₃ are identical or different and are fluorine, chlorine or hydrogen atoms or nitro groups.

3. The process as claimed in claim 1 or 2, which comprises employing as inert organic solvent an aromatic hydrocarbon, a chlorinated aromatic hydrocarbon or a mixture thereof.

4. The process as claimed in one or more of claims 1 to 3, which comprises employing as inert organic solvent cumene, mesitylene, cymene, diisopropylnaphthalene, a chlorotoluene and/or dichlorobenzene.

5. The process as claimed in one or more of claims 1 to 4, which comprises employing from 1 to 20, preferably from 2 to 5, parts by weight of solvent per part by weight of aromatic carboxylic acid.

6. The process as claimed in one or more of claims 1 to 5, which comprises employing from 0.8 to 1.5 mol, in particular from 1.0 to 1.3 mol, of urea per mole of aromatic carboxamide.

7. The process as claimed in one or more of claims 1 to 6, which comprises adding phosphorous acid as catalyst in the reaction in quantities of from 0.01 to 5, preferably from 0.05 to 2, % by weight.

8. The process as claimed in one or more of claims 1 to 7, which comprises carrying out the reaction at temperatures of from 140 to 200°C, in particular at temperatures of from 150 to 170°C.

9. The process as claimed in one or more of claims 1 to 8, which comprises employing instead of the pure solvent the mother liquor from a preceding batch.

10. The process as claimed in one or more of claims 1 to 9, which comprises recovering unreacted carboxylic acid and recycling it to the reaction.

## Revendications

1. Procédé pour la préparation d'amides des acides carboxyliques aromatiques de formule générale (I) 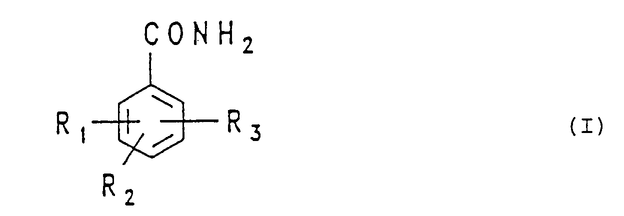 dans laquelle R₁, R₂ et R₃ sont identiques et différents et représentent l'hydrogène, des atomes de fluor, de chlore, de brome, des groupes alkyle en C₁-C₄, hydroxy ou nitro, ou R₁ et R₂ forment un cycle aromatique avec 5 ou 6 chaînons, lequel peut être substitué par des atomes de fluor, de chlore, de brome, par des groupes alkyle en C₁-C₄, hydroxy ou nitro, caractérisé en ce que l'on fait réagir les acides carboxyliques aromatiques de formule générale (II) 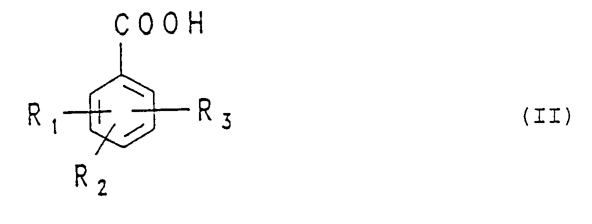 dans laquelle R₁, R₂, R₃ ont les significations précitées, dans un solvant organique inerte, avec l'urée, en ajoutant une quantité catalytique d'acide phosphoreux.

2. Procédé selon la revendication 1, caractérisé en ce que R₁, R₂ et R₃, identiques ou différents, représentent des atomes de fluor, de chlore, d'hydrogène ou des groupes nitro.

3. Procédé selon la revendication 1 ou 2, caractérisé en ce que l'on utilise en tant que solvant organique inerte un hydrocarbure aromatique, un hydrocarbure aromatique chloré ou leurs mélanges.

4. Procédé selon une ou plusieurs des revendications 1 à 3, caractérisé en ce que l'on utilise en tant que solvants organiques inertes le cumène, le mésitylène, le cymol, le diisopropylnaphtalène, un chlorotoluène et/ou dichlorobenzène.

5. Procédé selon une ou plusieurs des revendications 1 à 4, caractérisé en ce que l'on utilise pour une partie en poids d'acide carboxylique aromatique de 1 à 20, de préférence de 2 à 5 parties en poids de solvant.

6. Procédé selon une ou plusieurs des revendications 1 à 5, caractérisé en ce que l'on utilise par mole d'amide d'acide carboxylique aromatique de 0,8 à 1,5 mole, de préférence de 1,0 à 1,3 mole d'urée.

7. Procédé selon une ou plusieurs des revendications 1 à 6, caractérisé en ce que l'on ajoute à la réaction de l'acide phosphoreux dans des quantités de 0,01 à 5, de préférence de 0,05 à 2 % en poids, en tant que catalyseur.

8. Procédé selon une ou plusieurs des revendications 1 à 7, caractérisé en ce que l'on met en oeuvre la réaction à des températures de 140 à 200 °C, plus particulièrement à des températures de 150 à 170 °C.

9. Procédé selon une ou plusieurs des revendications 1 à 8, caractérisé en ce que l'on utilise, au lieu du solvant pur, la liqueur-mère provenant d'une charge précédente.

10. Procédé selon une ou plusieurs des revendications 1 à 9, caractérisé en ce que l'on récupère l'acide carboxylique n'ayant pas réagir et on le recycle dans la réaction.
